# EUROPÄISCHE PATENTSCHRIFT

(11) **EP 0 672 397 B1**
(45) Veröffentlichungstag und Bekanntmachung des Hinweises auf die Patenterteilung: **07.06.2000**
(21) Anmeldenummer: 94810159.7
(22) Anmeldetag: 15.03.1994
(51) Int. Cl.: A61F 2/38

(54) **Tibiaplateau für eine künstliches Kniegelenk**
Tibial plate for an artificial knee joint
Plateau tibial pour l'articulation artificielle de genou

(43) Veröffentlichungstag der Anmeldung: 20.09.1995
(73) Patentinhaber: Sulzer Orthopädie AG, 6340 Baar (CH)
(72) Erfinder: Schoch, Martin, CH-8143 Stallikon (CH); Godeau Denis, F-35800 Dinard (FR); Rolland Jean-Jacques, F-22100 Dinan (FR); Menou Patrick, F-35510 Cesson Sevigne (FR); Canciani Jean-Pierre,Dr., F-35132 Vezin le Coquet (FR); Feron Franck, F-35760 St Gregoire (FR); Delarue Patrice, F-22100 Dinan (FR); Triclot Philippe, F-35300 Fougeres (FR); Chaumont Guy, F-22100 Lehon (FR); Graf, Patrice, Dr., F-29200 Brest (FR)
(74) Vertreter: Trieblnig, Adolf

(56) Entgegenhaltungen:
- EP-A- 0 268 216
- EP-A- 0 306 744
- EP-A- 0 327 495
- FR-A- 2 141 126
- US-A- 4 207 627

## Beschreibung

Die Erfindung handelt von einem Tibiaplateau für ein künstliches Kniegelenk, umfassend eine metallische Plattform, welche mit Verankerungselementen an der Tibia verankerbar ist und zwei auf der Plattform medial und lateral von der Tibiaachse abgestützte Gleitflächen in einem Lagerkörper aus Kunststoff für gegenüberliegende Femurkondylen besitzt, wobei jede Gleitfläche auf einem individuellen Lagerkörper jeweils von anterior in die metallische Plattform einschiebbar ist und in dessen Endstellung mit dieser verklinkbar ist und wobei die metallische Plattform einen Einschnitt aufweist, welcher durch die Tibiaachse von posterior nach anterior verläuft und einer Tiefe > 75 % der totalen Abmessung des Tibiaplateaus in der Richtung des Einschnitts entspricht.

Die Patentanmeldung EP-A-0 306 744 offenbart eine metallische Plattform, bei welcher zwei metallische Schalen vom durch eine Brücke verbunden sind und einen Einschnitt von posterior bilden. Für den besseren Austausch von Lagerkörpern aus Kunststoff ist der vorstehende Rand der Metallschalen auf der Vorderseite reduziert. Statt einem überhöhten Rand ist eine Fixierklemme vorgesehen, die auf der Brücke aufsetzbar ist und mit quer gerichteten Zapfen die Lagerkörper in den Lagerschalen sichert.

In der französischen Patentanmeldung mit der Publikationsnummer FR 2 653 992 ist ein Tibiaplateau gezeigt, welches bei erhaltenem hinteren Kreuzband in ein Kniegelenk eingesetzt werden kann.

Ein Nachteil einer solchen Ausführungsform besteht darin, dass sie bei einem intakten vorderen Kreuzband aus Platzgründen nicht mehr anwendbar ist. Ganz allgemein ist das Einsetzen einer Tibiaplattform ein Platzproblem, wenn das hintere und das vordere Kreuzband erhalten bleiben sollen, da die Kreuzbänder nur begrenzt überdehnt werden können und eine Flexion und eine Verschiebung des Femurteils gegenüber dem Tibiateil nur im Rahmen der Bewegungsmöglichkeiten der Kreuzbänder liegen.

Aufgabe der vorliegenden Erfindung ist es, ein Tibiaplateau zu schaffen, das aufgrund seiner Form und Montagemöglichkeiten bei Kniegelenken mit intakten Kreuzbändern einsetzbar ist.

Diese Aufgabe wird mit den Kennzeichen des unabhängigen Anspruchs 1 gelöst, indem zur Erhaltung der Kreuzbänder der Einschnitt eine Breite von mehr als 15 mm aufweist und mit einer Winkelabweichung von 3° bis 12° aus der sagittalen Richtung gegen medial gerichtet ist.

Diese Anordnung hat den Vorteil, dass an der Tibia genügend Material im Bereich der Kreuzbänder erhalten bleibt, womit die Bandstrukturen geschont werden und die tibiale Abstützfläche möglichst gross gehalten wird. Durch die Schrägstellung wird der Einschnitt nicht unnötig breit gemacht, was einer Vergrösserung der Gleitflächen zugute kommt. Trotzdem bleibt zwischen den beiden Hälften der metallischen Plattform ein steifer Steg anterior stehen, welcher der Plattform bis zu ihrer endgültigen Verankerung genügend Festigkeit verleiht.

Weitere vorteilhafte Ausgestaltungen der Erfindung sind in den abhängigen Ansprüchen 2 bis 10 gezeigt. So bewirken ortsfeste Verankerungselemente, die mit der Unterseite der metallischen Plattform ortsfest posterior verbunden sind, dass Einpresskräfte zum Einpressen der ortsfesten Verankerungselemente in der Richtung der Tibiaachse nur am Rand der metallischen Plattform aufgebracht werden müssen, wo Werkzeuge ansetzbar sind, während anterior bewegliche Verankerungselemente nach dem Aufbringen der metallischen Plattform an der Tibia wegen ihrer Nähe zum vorderen Rand durch Einsetzen von oben befestigbar sind. Damit ist die metallische Plattform in und auf einer Ebene quer zur Tibiaachse fixierbar.

Ein weiterer Vorteil ergibt sich für die Belastbarkeit, wenn die Gleitfläche und die metallische Plattform - wie bei der Bandbelastung im natürlichen Kniegelenk - auf der medialen Seite eine grössere Abmessung von posterior nach anterior aufweisen.

Die Gleitflächen der Lagerkörper bilden eine Ebene, die durch unterschiedliche Lagerkörper in ihrer Höhe und in ihrer Neigung zur metallischen Plattform variiert werden kann. Wenn die Lagerkörper mit einer lösbaren Verklinkung auswechselbar sind, kann nach dem Fixieren der metallischen Plattform an der Tibia und von Femurkondylen am Femur ein Feinabgleich für die Lage der Gleitebene vorgenommen werden und die passenden Lagerkörper können bestimmt werden. Je nach Zustand von den Bändern werden für intakte Seitenbänder zylindrische Kondylen und eine Gleitebene oder bei geschwächten Bändern sphärische Kondylen und Lagerkörper mit flachen Führungsrinnen von anterior nach posterior eingesetzt, um eine zusätzliche Führungshilfe zu geben. Die Rinnen können an ihrem Auslauf posterior und/oder anterior überhöht sein, um mehr Sicherheit in Endlagen zu vermitteln.

Wegen der Auswahlmöglichkeiten ist es vorteilhaft, wenn die passenden Lagerkörper jeweils paarweise durch einen elastischen Steg verbunden sind. Dies verhindert Verwechslungen. Dabei ist der Steg mit Vorteil so gestaltet, dass während dem Einschieben der Lagerkörper kleine Relativbewegungen zueinander möglich sind. Dieser Steg kann selbst zur Verklinkung der beiden Lagerkörper verwendet werden, wenn er zum Beispiel als biegeelastischer Draht zwischen den Lagerkörpern ausgeführt ist, was den Vorteil hat, dass keine federnden Klinken im Kunststoff der Lagerkörper notwendig sind.

Als ortsfeste Befestigungsmittel auf der Unterseite der metallischen Plattform sind Dorne oder Schneidhülsen von Vorteil, die nach dem Aufsetzen der metallischen Plattform die relative Steifigkeit ihrer beiden Hälften zueinander verstärken. Dabei ist es vorteilhaft, wenn diese Befestigungsmittel mehr als 3 mm vorstehen, um wirksame Verankerungsflächen zu schaffen. Sie sind jedoch weniger hoch als die Summe der Dicke des Femurimplantates an den dorsalen Kondylen und die Höhe des über die metallische Plattform ragenden Lagerkörpers, um das Einfahren der metallischen Plattform im Bereich der Kreuzbänder möglich zu machen.

Angesenkte Bohrungen in der metallischen Plattform sind mit Vorteil soweit gegen anterior versetzt, dass bei eingepresster Plattform und abgewinkeltem Oberschenkel die Kondylen soweit nach posterior verschiebbar sind, dass bewegliche Verankerungselemente zum Beispiel in Form von Befestigungsschrauben von oben einsetzbar sind.

Ein Kniegelenk mit dem beschriebenen Tibiaplateau kommt mit seinem Bewegungsspielraum sehr nah an ein natürliches Gelenk, bei dem der Meniskus entfernt werden musste.

Im folgenden wird die Erfindung anhand von einem Beispiel beschrieben. Es zeigen:
- Fig. 1: Schematisch die Draufsicht auf eine metallische Plattform;
- Fig. 2: schematisch die Vorderansicht einer metallischen Plattform gemäss Fig. 1 mit eingesetzten Lagerkörpern;
- Fig. 3: schematisch einen Schnitt durch Lagerkörper gemäss Fig. 2, die als Gleitfläche jeweils eine flache Rinne aufweisen;
- Fig. 4: schematisch einen Längsschnitt durch eine metallische Plattform gemäss Fig. 1 mit einem Lagerkörper;
- Fig. 5: schematisch einen Ausschnitt von einer metallischen Plattform mit einem Verankerungselement, das als Dorn ausgeführt ist;
- Fig. 6: schematisch die Draufsicht auf ein Tibiaplateau, dessen beide Lagerkörper mit einem elastischen Steg verbunden sind; und
- Fig. 7: schematisch einen vergrösserten Längsschnitt durch Figur 6.

In den Figuren wird für ein künstliches Kniegelenk ein Tibiaplateau gezeigt, welches eine metallische Plattform umfasst, die mit Verankerungselementen an der Tibia verankerbar ist, sowie zwei auf der Plattform medial und lateral von der Tibiaachse abgestützte Gleitflächen in einem Lagerkörper aus Kunststoff besitzt. Zur Erhaltung von Kreuzbändern ist an der metallischen Plattform ein Einschnitt von mehr als 15 mm Breite von posterior gegen anterior angebracht, welcher durch die Tibiaachse verläuft, aus der sagittalen Richtung eine Winkelabweichung von 3° bis 12° gegen medial aufweist und eine Tiefe von grösser/gleich 75 % der totalen Abmessung des Tibiaplateaus in der Richtung des Einschnitts besitzt. Zur Platzeinsparung beim Einsetzen der metallischen Plattform sind die Lagerkörper entfernt, welche nachträglich von anterior in die Plattform einschiebbar und mit dieser verklinkbar sind.

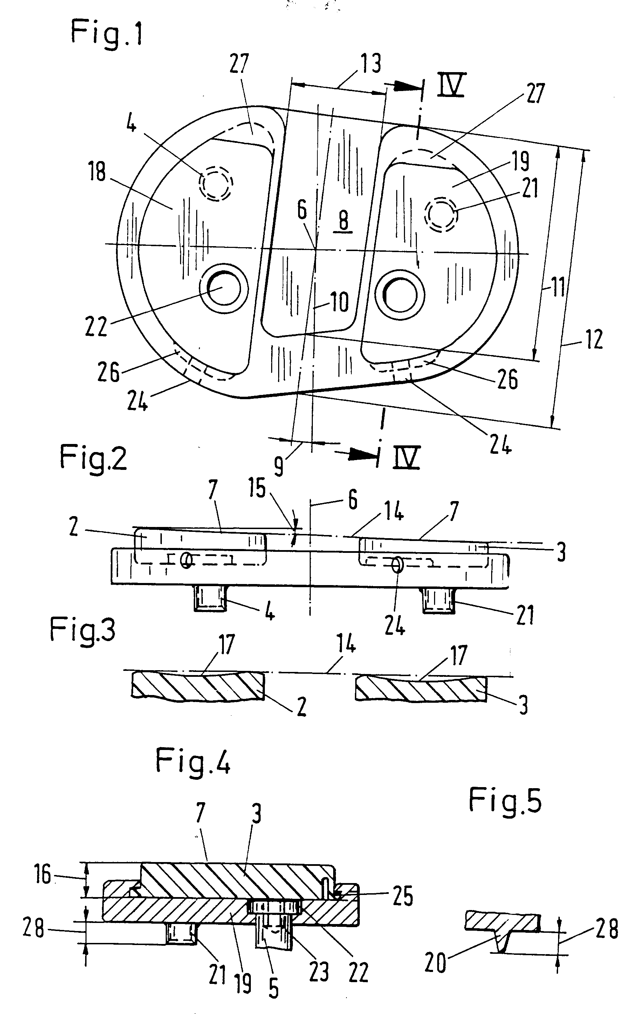

Die metallische Plattform in den Figuren 1 und 2 besteht aus einem medialen Teil 18 und einem lateralen Teil 19, die durch einen Einschnitt 8 getrennt sind und anterior über ein festes Joch miteinander verbunden sind. Der Einschnitt verläuft durch die Tibiaachse 6 und ist um eine Winkelabweichung 9 aus der sagittalen Richtung 10 gegen medial gerichtet. Seine Breite 13 ist grösser als 15 mm. Die Winkelabweichung 9 liegt in einem Bereich von 3° bis 12°, wobei 7° ein bevorzugter und häufiger Wert ist. Im medialen und lateralen Teil 18, 19 ist jeweils eine Vertiefung eingelassen, in die ein Lagerkörper 2, 3, der eine obere Gleitfläche 7 besitzt, einbringbar ist. Dazu wird der Lagerkörper 2, 3 von anterior über den Rand der metallischen Plattform 1 gegen posterior eingeschoben, bis er posterior in einer Tasche 27 gehalten ist und sich anterior in die Vertiefung der Plattform einpressen lässt, bis er mit einer Klinke 25 in einem Rücksprung 26 der Plattform einrastet. Die Klinke 25 kann durch eine Oeffnung 24 im Rand der Plattform mit einem Hilfswerkzeug wieder gelöst werden. Die Tiefe 11 vom Einschnitt 8 beträgt mehr als 75 % der Abmessung 12 der Plattform in Richtung des Einschnitts.

Die beiden Gleitflächen 7 der Lagerkörper 2 und 3 liegen in einer Ebene 14, welche eine Neigung 15 aufweist, um zwischen der Auflagefläche für die metallische Plattform und der Ebene 14 eine Keilform zu erzeugen. An der Unterseite der Plattform 1 sind posterior starre Verankerungselemente 4 in Form von Schneidhülsen 21 befestigt. Den gleichen Zweck erfüllen auch Befestigungsdorne 20 aus Figur 5; die sich wie die Schneidhülsen in einer vorgesehenen Resektionsfläche der Tibia einpressen lassen, wenn man am Rand der Plattform 1 Kräfte in der Richtung der Tibiaachse 6 aufbringt. Auf der Hälfte gegen anterior sind an der Plattform Befestigungsbohrungen 22 angebracht, in die bewegliche Verankerungselemente 5 zum Beispiel Befestigungsschrauben 23 von oben eingesetzt werden können.

In Figur 3 sind Lagerkörper 2, 3 gezeigt, die jeweils eine flache Rinne 17 von anterior nach posterior aufweisen.

In Figur 4 ist eine Schneidhülse 21 gezeigt, deren Höhe 28 über die Auflagefläche der metallischen Plattform 1 kleiner ist als die Summe der Höhe des Lagerkörpers, die die metallische Plattform überragt und die Dicke des entsprechenden Femurimplantates an den dorsalen Kondylen, jedoch grösser als 3 mm. Solange die Lagerkörper entfernt sind und das Femurteil noch nicht eingebracht ist, lässt sich die Plattform 1 mit der Schneidhülse zwischen Resektionsfläche der Tibia und Femurkondylen von anterior einschieben und setzen. Anschliessend können die Befestigungsschrauben 23 in der vorderen Hälfte angebracht werden und die Lagerkörper von posterior eingeschoben und mit den Klinken 25 gesichert werden. Dabei können die Lagerkörper 2, 3 in Höhe und Neigung variiert werden, um eine Feinkorrektur für die Lage der Femurkondylen vorzunehmen.

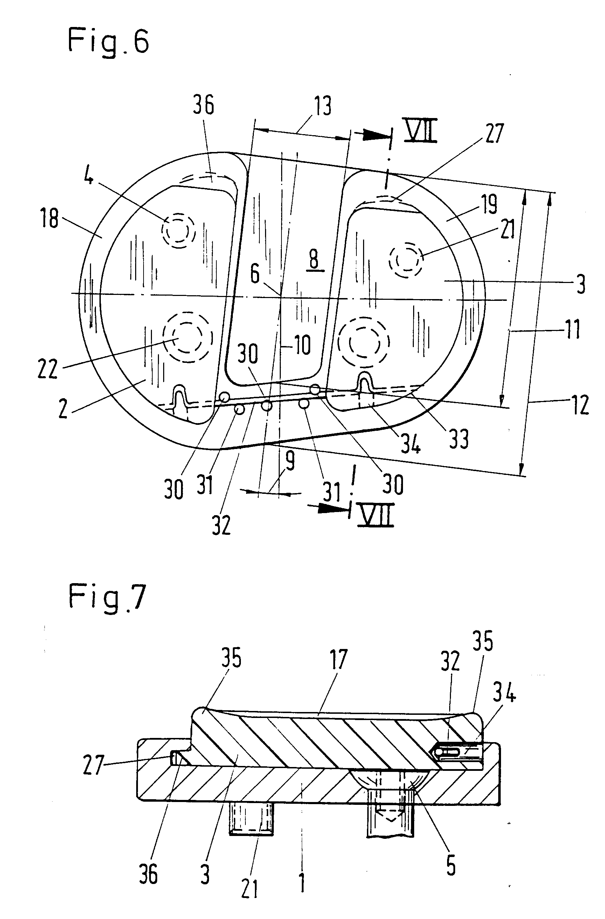

In Figur 7 ist die Rinne 17 an ihrem Auslauf posterior und anterior mit einer Ueberhöhung 35 versehen, um die Wegbegrenzung der Bänder zu unterstützen.

In den Figuren 6 und 7 ist eine weitere Art der Verklinkung mit einem Verbindungssteg 32 zwischen den Lagerkörpern 2, 3 aufgeführt. Die Lagerkörper sind durch einen Steg 32 in Form von einem biegeelastischen Draht verbunden. Der in Führungsbohrungen 33 in den Lagerkörpern 2, 3 eingezogene Draht ist mit einem Buckel in einer Querbohrung 34 verstemmt. Die beiden zueinandergehörenden Lagerkörper 2, 3 sind locker miteinander verbunden und können nicht mit falschen Lagerkörpern kombiniert werden. Die beiden Lagerkörper 2, 3 lassen sich gemeinsam annähernd horizontal einschieben bis ihre Zungen 36 posterior in den Taschen 27 gefangen sind. In dieser Lage werden Lagerkörper und Draht 32 abwärts gedrückt bis der biegeelastische Draht 32 in einem Kanal 29 über Nocken 30 hinweggedrückt ist und einrastet, um die Lagerkörper zu sichern. Ueber Aushebeöffnungen 31 kann der Draht 32 mit einem spitzen Hilfswerkzeug wieder gelöst werden.

## Patentansprüche

1. Tibiaplateau für ein künstliches Kniegelenk, umfassend eine metallische Plattform (1), welche mit Verankerungselementen (4, 5) an der Tibia verankerbar ist und zwei auf der Plattform (1) medial und lateral von der Tibiaachse (6) abgestützte Gleitflächen (7) in einem Lagerkörper (2, 3) aus Kunststoff für gegenüberliegende Femurkondylen besitzt, wobei jede Gleitfläche (7) auf einem individuellen Lagerkörper (2, 3) jeweils von anterior in die metallische Plattform (1) einschiebbar ist und in dessen Endstellung mit dieser verklinkbar ist und wobei die metallische Plattform (1) einen Einschnitt (8) aufweist, welcher durch die Tibiaachse (6) von posterior nach anterior verläuft und einer Tiefe (11) > 75 % der totalen Abmessung (12) des Tibiaplateaus in der Richtung des Einschnitts (8) entspricht, dadurch gekennzeichnet, dass zur Erhaltung der Kreuzbänder der Einschnitt (8) eine Breite (13) von mehr als 15 mm aufweist und mit einer Winkelabweichung (9) von 3° bis 12° aus der sagittalen Richtung (10) gegen medial gerichtet ist.

2. Tibiaplateau nach Anspruch 1, dadurch gekennzeichnet, dass die metallische Plattform posterior auf ihrer Unterseite ortsfeste Verankerungselemente (4) aufweist, während sie anterior bewegliche Verankerungselemente (5) aufweist, welche nach dem Aufbringen der metallischen Plattform auf die Tibia einsetzbar sind.

3. Tibiaplateau nach Anspruch 1 oder 2, dadurch gekennzeichnet, dass der Lagerkörper (2) und die metallische Plattform (18) auf der medialen Seite eine grössere Abmessung von posterior nach anterior aufweisen als auf der lateralen Seite.

4. Tibiaplateau nach einem der Ansprüche 1 bis 3, dadurch gekennzeichnet, dass die beiden Gleitflächen (7) der Lagerkörper (2, 3) in einer Ebene (14) liegen, die durch die Verwendung unterschiedlicher Lagerkörper (2, 3) gegenüber der metallischen Plattform (1) in ihrer Höhe (16) und/oder in eine von der Parallelität abweichende Neigung (15) veränderbar ist.

5. Tibiaplateau nach Anspruch 4, dadurch gekennzeichnet, dass jede der Gleitflächen (7) als eine von anterior nach posterior verlaufende flache Rinne (17) ausgebildet ist.

6. Tibiaplateau nach Anspruch 5, dadurch gekennzeichnet, dass die Gleitfläche (7) posterior und/oder anterior eine Ueberhöhung (35) aufweisen.

7. Tibiaplateau nach einem der Ansprüche 1 bis 6, dadurch gekennzeichnet, dass die beiden Lagerkörper (2, 3) durch einen elastischen Steg (32) verbunden sind, um eine gemeinsame Handhabung zu ermöglichen und um Verwechslungen auszuschliessen.

8. Tibiaplateau nach Anspruch 7, dadurch gekennzeichnet, dass der Steg (32) zum Beispiel in Form von einem Draht der Verklinkung der beiden Lagerkörper (2, 3) in ihrer Endstellung dient.

9. Tibiaplateau nach einem der Ansprüche 2 bis 8, dadurch gekennzeichnet, dass der durch den Einschnitt (8) getrennte mediale Teil (18) und laterale Teil (19) der metallischen Plattform (1) auf seiner Unterseite posterior einen Befestigungsdorn (20) oder eine Schneidhülse (21) aufweist, während er anterior eine angesenkte Bohrung (22) für ein einsetzbares Verankerungselement (5), zum Beispiel für eine Befestigungsschraube (23), aufweist.

10. Tibiaplateau nach einem der Ansprüche 1 bis 9, dadurch gekennzeichnet, dass die fest mit der metallischen Plattform (1) verbundenen Verankerungselemente (4, 20, 21) in Richtung der Tibia um eine Höhe (28) vorstehen, welche grösser als 3 mm ist und welche kleiner als die Summe der Dicke des Femurimplantates an den dorsalen Kondylen und die Höhe des über die metallische Plattform ragenden Lagerkörpers ist, um ein Einfahren der metallischen Plattform im Bereich der Kreuzbänder möglich zu machen.

11. Künstliches Kniegelenk mit einem Tibiaplateau nach einem der Ansprüche 1 bis 10.

## Claims

1. A tibia plate for an artificial knee joint, comprising a metal platform (1), which can be attached to the tibia by anchorage members (4, 5) and possesses two slide faces (7) supported on the platform (1) medially and laterally from the tibia axis (6) in a bearing member (2, 3) made from plastics material for opposite femur condyles, wherein each slide face (7) on an individual bearing member (2, 3) can be inserted from the anterior side into the metal platform (1) and in its final position can be latched therewith and wherein the metal platform (1) has a notch (8), which extends through the tibia axis (6) from the posterior to the anterior side and corresponds to a depth (11) > 75 % of the total dimension (12) of the tibia plate in the direction of the notch (8),
**characterised in that** to retain cruciate ligaments the notch (8) has a width (13) of more than 15 mm and with an angular deviation (9) of 3° to 12° from the sagittal direction (10) is directed medially.

2. A tibia plate according to Claim 1,
**characterised in that** at the posterior side the metal platform has anchorage members (4) fixed to its underside, whereas at the anterior side it has moveable anchorage members (5) which can be inserted after the metal platform has been placed on the tibia.

3. A tibia plate according to Claim 1 or 2,
**characterised in that** on the medial side the bearing member (2) and the metal platform (18) have a larger dimension from the posterior to the anterior side than on the lateral side.

4. A tibia plate according to one of Claims 1 to 3, **characterised in that** the two slide faces (7) of the bearing members (2, 3) lie in a plane (14), which, by using different bearing members (2, 3), can be altered in relation to the metal platform (1) in its height (16) and/or in an inclination (15) deviating from the parallelism.

5. A tibia plate according to Claim 4,
**characterised in that** each of the slide faces (7) is constructed as a flat groove (17) extending from the anterior side to the posterior side.

6. A tibia plate according to Claim 5,
**characterised in that** the slide face (7) has a raised part (35) at the posterior and/or anterior side.

7. A tibia plate according to one of Claims 1 to 6,
**characterised in that** the two bearing members (2, 3) are connected by an elastic web (32) to enable their joint manipulation and to prevent confusion.

8. A tibia plate according to Claim 7,
**characterised in that** the web (32), in the form of a wire for example, is used to latch the two bearing members (2, 3) in their final position.

9. A tibia plate according to one of Claims 2 to 8,
**characterised in that** the medial part (18) and lateral part (19) of the metal platform (1) separated by the notch (8) on their underside have an attachment spike (20) or a cutting sleeve (21) at the posterior side, while at the anterior side it has a countersunk bore (22) for an insertable anchorage member (5), for example for a fastening screw (23).

10. A tibia plate according to one of Claims 1 to 9, **characterised in that** the anchorage members (4, 20, 21) securely connected to the metal platform (1) protrude in the direction of the tibia by a height (28), which is greater than 3 mm and which is less than the sum of the thickness of the femoral implant at the dorsal condyles and the height of the bearing member protruding above the metal platform, in order to enable the metal platform to be introduced in the region of the cruciate ligaments.

11. An artificial knee joint having a tibia plate according to one of Claims 1 to 10.

## Revendications

1. Plateau tibial pour une articulation artificielle du genou, comportant une plate-forme métallique (1) qui peut être ancrée par des éléments d'ancrage (4, 5) au tibia et qui possède deux faces de glissement (7) supportées sur la plate-forme (1) au côté médial et latéral de l'axe de tibia (6), dans un corps de palier (2, 3) en matière synthétique pour des condyles de fémur opposés, où chaque face de glissement (7) peut être insérée sur un corps de palier individuel (2, 3) respectivement depuis le côté antérieur dans la plate-forme métallique (1) et, dans sa position d'extrémité, peut être enclenchée dans celle-ci, et où la plate-forme métallique (1) présente une entaille (8) qui s'étend à travers l'axe du tibia (6) du côté postérieur vers le côté antérieur et qui correspond à une profondeur (11) > 75° de la dimension totale (12) du plateau tibial en direction de l'entaille (8), caractérisé en ce que pour la conservation des ligaments croisés, l'entaille (8) a une largeur (13) supérieure à 15 mm et est orientée avec un écart angulaire (9) de 3° à 12° de la direction sagittale (10) vers le côté médial.

2. Plateau tibial selon la revendication 1, caractérisé en ce que la plate-forme métallique présente postérieurement sur son côté inférieur des éléments d'ancrage fixes (4) tandis qu'elle présente antérieurement des éléments d'ancrage mobiles (5) qui, après l'application de la plate-forme métallique, peuvent être placés dans le tibia.

3. Plateau tibial selon la revendication 1 ou 12, caractérisé en ce que le corps de palier (2) et la plate-forme métallique (18) ont sur le côté médial une plus grande dimension du côté postérieur vers le côté antérieur que sur le côté latéral.

4. Plateau tibial selon l'une des revendications 1 à 3, caractérisé en ce que les deux faces de glissement (7) des corps de palier (2, 3) se situent dans un plan (14) qui, par l'utilisation de corps de palier différents (2, 3), peuvent être modifiées par rapport à la plate-forme métallique (1) en hauteur (16) et/ou selon une inclinaison (15) s'écartant de la parallélité.

5. Plateau tibial selon la revendication 4, caractérisé en ce que chacune des faces de glissement (7) est réalisée comme rainure plate (17) s'étendant du côté antérieur vers le côté postérieur.

6. Plateau tibial selon la revendication 5, caractérisé en ce que les faces de glissement (7) présentent postérieurement et/ou antérieurement une surélévation (35).

7. Plateau tibial selon l'une des revendications 1 à 6, caractérisé en ce que les deux corps de palier (2, 3) sont reliés par une barrette élastique (32) pour permettre une manipulation commune et pour éviter des confusions.

8. Plateau tibial selon la revendication 7, caractérisé en ce que la barrette (32), par exemple sous la forme d'un fil, sert à l'enclenchement des deux corps de palier (2, 3) dans leur position finale.

9. Plateau tibial selon l'une des revendications 2 à 8, caractérisé en ce que la partie médiale (18) séparée par l'entaille (8) et la partie latérale (19) de la plate-forme métallique (1) présente sur son côté inférieure postérieurement un tenon de fixation (20) ou une douille tranchante (21) tandis qu'elle présenta antérieurement un perçage chanfreiné (22) pour un élément d'ancrage insérable (5), par exemple pour une vis de fixation (23).

10. Plateau tibial selon l'une des revendications 1 à 9, caractérisé en ce que les éléments d'ancrage (4, 20, 21) reliés solidement à la plate-forme métallique (1) font saillie en direction du tibia selon une hauteur (28) qui est plus grande que 3 mm et qui est plus petite que la somme de l'épaisseur de l'implant de fémur aux condyles dorsaux et la hauteur du corps de palier faisant salle sur la plate-forme métallique pour permettre une insertion de la plate-forme métallique au voisinage des ligaments croisés.

11. Articulation artificielle du genou comportant un plateau tibial selon l'une des revendications 1 à 10.
